# FASCICULE DE BREVET EUROPEEN

(11) **EP 2 790 664 B1**
(45) Date de publication et mention de la délivrance du brevet: **26.07.2017**
(21) Numéro de dépôt: 12798325.2
(22) Date de dépôt: 17.12.2012
(51) Int. Cl.: A61K 8/85, A61Q 3/02

(54) **COMPOSITION COSMÉTIQUE ANHYDRE APPLICABLE SUR LES ONGLES**
WASSERFREIE KOSMETISCHE ZUSAMMENSETZUNG ZUM AUFTRAGEN AUF NÄGEL
ANHYDROUS COSMETIC COMPOSITION APPLICABLE TO THE NAILS

(30) Priorité: 16.12.2011 LU 91918
(43) Date de publication de la demande: 22.10.2014
(73) Titulaire: International Lacquers S.A., 3225 Bettembourg (LU)
(72) Inventeur: WIMMER, Eric, L-1213 Luxembourg (LU); DEROSIER, Frédéric, F-57680 Corny Sur Moselle (FR); IGNACCOLO, Aurélie, F-57700 Neufchef (FR); DELAS, Christophe, L-3926 Mondercange (LU)
(74) Mandataire: Office Freylinger
(86) Numéro de dépôt international: PCT/EP2012/075086
(87) Numéro de publication internationale: WO 2013/087624

(56) Documents cités:
- EP-A1- 2 077 139
- EP-A2- 2 353 578
- Martina Heldemann (Inolex): "Colour Cosmetics: Castor oil alternative for lipstick formulas", Cossma Cossma, 5 septembre 2011 (2011-09-05), XP002682737, Extrait de l'Internet: URL:http://www.cossma.com/en/news/single-v iew/artikel/colour-cosmetics-castor-oil-al ternative-for-lipstick-formulas.html [extrait le 2012-09-03]

## Description

### Domaine technique

La présente invention concerne une composition cosmétique anhydre applicable sur les ongles et qui forme un film dur, brillant et tenace.

### Etat de la technique

Les différentes propriétés que l'on demande à un vernis à ongles sont les suivantes :
- Bonne application, avec un pinceau de taille et forme adéquate. Le pinceau permet d'obtenir une surface lisse, sans stries avec une épaisseur constante afin d'obtenir un film et une coloration homogènes.
- Un temps de séchage approprié de l'ordre de quelques minutes.
- Une tenue sur l'ongle dans le temps, que l'on définit par la tenue en évitant l'écaillement du vernis ou son usure en bout d'ongle.
- Une brillance importante à l'application, c'est à dire quelques minutes après séchage.
- Une tenue de la brillance dans le temps, c'est à dire une brillance diminuant le moins possible dans les jours qui suivent l'application.

Le paramètre d'application est plus particulièrement en relation avec la rhéologie du milieu. La viscosité de la formulation dépend principalement des différents ingrédients utilisés dans la formulation et de leur dosage au sein de cette formulation. L'application peut également dépendre des matières colorantes utilisées dans la formulation colorée mais aussi de la qualité de l'applicateur.

Le temps de séchage est principalement dépendant des solvants utilisés dans la formulation et en particulier leur température d'ébullition et leur tension de vapeur. L'utilisation de différents solvants ayant des propriétés physico-chimiques différentes permet de moduler le temps de séchage et de durcissement du film qui peut également dépendre des ingrédients utilisés. D'autres ingrédients comme les résines et leur quantité utilisée dans la formulation peuvent influencer le temps de séchage.

La durabilité du vernis sur l'ongle ou tenue est principalement due aux agents non volatils de la formulation tels que les résines, la nitrocellulose ou les différents additifs annexes.

La brillance est apportée par les différents constituants non volatils du vernis à ongles comme les résines ou la nitrocellulose. Toutefois, ce paramètre est fortement affecté par les composés solides non solubles compris dans la formulation. Parmi ces composés solides non solubles, on citera notamment les pigments qui entraînent par leurs caractéristiques physico-chimiques une diminution de la brillance par rapport à la même formulation non pigmentée. Malgré les différents procédés engagés lors de la préparation de ces pigments, une diminution de la brillance est clairement notée. Les différentes étapes mises en oeuvre dans la préparation des pigments avant leur utilisation dans le vernis à ongles permettent plusieurs actions. Ces actions sur les pigments sont les suivantes :
- Le mouillage, qui permet de déplacer et d'éliminer l'air ou l'humidité qui se trouve à la surface du pigment.
- La dispersion qui permet de :
   - Casser les agglomérats et les agrégats de pigments ; les agglomérats correspondent à l'association de structures primaires de pigments par des contacts de surface, les agrégats correspondent plutôt à une structure secondaire ayant pour origine l'association des structures primaires par des contacts bord à bord
   - Distribuer de façon homogène les particules primaires des pigments afin d'éviter la reformation des structures secondaires
- La stabilisation de la dispersion qui évite le retour des agrégats.

A ces différentes actions sur le pigment lui-même, s'ajoute l'utilisation d'ingrédients annexes utilisés en faible quantité, mais dont le pourcentage d'utilisation dépend directement de la nature (organique ou non) du pigment à formuler. Ces ingrédients appelés mouillants ou dispersants ont en général des structures bifonctionnelles. L'une des fonctions, ayant une forte affinité pour la surface du pigment, s'oriente vers celle-ci et se lie à elle par des liaisons chimiques ou des liaisons d'énergie plus faible de type Van der Waals. La seconde fonction s'oriente quant à elle vers la phase continue, composée généralement de solvants et de résines.

Ces mouillants assurent ainsi une meilleure affinité des pigments vers le milieu organique et évite également la reformation des structures secondaires décrites plus haut. La reformation de ces structures secondaires peut en effet se révéler problématique d'un point de vue colorimétrique mais peut aussi conduire à une instabilité de la formule caractérisée notamment par une sédimentation des pigments, les structures secondaires étant plus lourdes et plus volumineuses que les structures primaires.

Une grande variété de mouillants/dispersants sont connus de l'homme de l'art, que ce soit dans le milieu cosmétique ou dans le milieu de la peinture. Ces mouillants/dispersants peuvent se présenter sous la forme de composés simples ou encore sous la forme de composés polymériques ayant des chaînes latérales fonctionnalisées.

On notera notamment le brevet US 4,302,442 qui décrit l'utilisation de phosphatide comme la lécithine pour empêcher la migration des pigments, la lécithine est généralement utilisée en association avec un acide organique ou inorganique.

On citera également le brevet 5,133,966 qui décrit l'utilisation d'acides gras et leurs sels dans la formulation pour améliorer la dispersion des pigments en association avec une résine dite « carrier resin ».

Même si ces documents montrent que l'utilisation de différents dérivés permet une amélioration accrue de la stabilité des pigments dans la formulation empêchant ainsi la réagglomération, les phénomènes de flottation ou les phénomènes de sédimentation, rien n'est mentionné concernant la brillance de la formulation colorée finale.
EP 2 077 139 A1 décrit une composition cosmétique anhydre pour vernis à ongles comprenant au moins un solvant organique cosmétiquement acceptable et une quantité efficace d'au moins un agent d'amélioration de la brillance qui est un dérivé de pentaérythritol choisi parmi les tétraesters d'acide gras de pentaérythrityle.

De plus, au vu de la grande variété d'ingrédients utilisés et utilisables dans ce genre de compositions, il existe un réel besoin de mieux maîtriser les résultats obtenus, en particulier bien sûr en ce qui concerne la brillance. En effet, pour le consommateur cette caractéristique est un critère important puisque la brillance affecte directement l'appréciation esthétique générale de la composition.

### Objet de l'invention

Un objet de la présente invention est par conséquent de proposer des formulations du type indiqué ci-dessus, mais présentant une brillance augmentée et de préférence plus durable dans le temps par rapport aux formulations connues.

Conformément à l'invention, cet objectif est atteint par une composition selon la revendication 1, respectivement une utilisation selon la revendication 6.

### Description générale de l'invention

Afin de résoudre le problème mentionné ci-dessus, la présente invention propose une composition cosmétique anhydre, notamment pour vernis à ongles ou de soins des ongles, comprenant au moins un solvant organique cosmétiquement acceptable, au moins un agent filmogène dérivé de la cellulose et une quantité efficace d'au moins un agent d'amélioration de la brillance, dans laquelle le ou les agent(s) d'amélioration de la brillance est/sont un copolymère obtenu par condensation d'acide adipique et de pentaérythritol comprenant des groupes terminaux d'acide caproïque, d'acide heptanoïque, d'acide caprylique et/ou d'acide caprique ; ou un mélange de plusieurs de ces copolymères.

En effet, les inventeurs ont découvert d'une manière surprenante que lesdits copolymères par condensation d'acide adipique et de pentaérythritol permettent de réaliser l'objectif d'augmenter nettement la brillance de compositions cosmétiques. De plus, cet effet est non seulement immédiat, c'est-à-dire directement obtenu à l'application, mais il est également significativement plus durable dans le temps.

Les copolymères utilisables sont ceux pouvant être obtenus par condensation d'acide adipique et de pentaérythritol d'une part et par addition de groupes terminaux choisis parmi l'acide caproïque, l'acide heptanoïque, l'acide caprylique, l'acide caprique, ou l'une quelconque des combinaisons de ces acides d'autre part. En général, ces copolymères possèdent une viscosité à 25°C de l'ordre de 700 mPa.s, par exemple de 600 à 800 mPa.s, de manière préférée entre 650 et 750 mPa.s. De préférence, le copolymère est un copolymère d'acide adipique et de pentaérythritol à groupes terminaux d'acide caproïque, d'acide heptanoïque, d'acide caprylique et d'acide caprique, c'est-à-dire le polyester-4 (désignation INCI).

Par l'expression « quantité efficace d'au moins un agent d'amélioration de la brillance », on entend une quantité suffisante pour obtenir une amélioration notable et significative de la brillance après application de la composition cosmétique. Cette quantité minimale en agent d'amélioration de la brillance à mettre en oeuvre, qui peut varier selon la nature du solvant cosmétiquement acceptable retenu pour la composition, ainsi qu'en fonction des autres ingrédients choisis, peut être déterminée sans aucune difficulté au moyen d'un test classique de mesure de la brillance, tel que celui donné dans les exemples ci-après. En pratique, le ou les agent(s) d'amélioration de la brillance représente(nt) en général 0.01 à 25 % en poids, de préférence 0.1 à 20 % en poids, en particulier de 0.25 à 15 % en poids de la composition totale.

Comme déjà mentionné, le premier avantage de la présente invention consistant à proposer l'utilisation d'un ou de plusieurs copolymères obtenus par condensation d'acide adipique et de pentaérythritol comprenant des groupes terminaux d'acide caproïque, d'acide heptanoïque, d'acide caprylique et/ou d'acide caprique est l'accroissement significatif de la brillance de la formulation dès l'application, c'est-à-dire que, contrairement à d'autres agents connus, la brillance est directement améliorée. Le deuxième avantage est que l'effet de brillance est plus durable que pour de nombreux agents connus. Il en résulte le double effet d'un taux de brillance plus important au départ, combiné à une constance élevée de la brillance pendant plusieurs jours après application.

D'autre part, l'ajout des agents d'amélioration de la brillance selon l'invention n'affecte pas négativement les autres qualités des compositions, à savoir les bonnes caractéristiques d'application et le temps de séchage.

Un avantage additionnel important de la présente invention est que les compositions cosmétiques anhydre, de préférence les formulations de vernis à ongles, à brillance améliorée peuvent comprendre les ingrédients habituellement utilisés par l'homme de l'art. L'homme de métier n'est donc pas obligé de reconsidérer tous les ingrédients et leurs effets, s'il souhaite utiliser les présents agents d'améliorations de la brillance. Les présents agents d'amélioration de la brillance présentent par conséquent une très bonne compatibilité tant avec les solvants, qu'avec les ingrédients courants dans le domaine.

Les solvants utilisables dans l'invention sont choisis par exemple parmi les suivants : l'acétate de butyle, l'acétate d'isobutyle, l'acétate de propyle, l'acétate de méthyle, l'acétate d'éthyle, le toluène, l'isopropanol, le diacétone alcool, l'éthanol dénaturé (avec la méthyléthylcétone ou le diéthyléther), le n-butanol, les hydrocarbures linéaires ou cycliques, comme l'hexane, le cyclohexane, l'heptane, l'isododécane. On citera également les dérivés de paraffine de type Isopar qui sont des dérivés paraffiniques qui ont des chaînes carbonées de taille variable, ainsi que les glycols comme l'éthylène glycol, le propylène glycol, le dipropylène glycol ou le tripropylène glycol.

Les solvants ou leurs mélanges peuvent être utilisés entre 5 et 80 % en poids de la formulation totale. Ils sont sélectionnés selon leurs propriétés physico-chimiques (température d'ébullition par exemple) et leur aptitude à solubiliser les autres constituants de la formulation. Ils seront également sélectionnés selon leurs propriétés organoleptiques (odeur principalement) et leurs possibles utilisations selon les réglementations en vigueur.

La composition selon l'invention pourra comprendre en outre de 0.1 à 10 % en poids, de préférence 0.2 à 8 % en poids, en particulier de 0.25 à 7,5 % en poids de la composition totale d'une ou de plusieurs matières colorantes ou agents de coloration, comme les pigments et les particules nacrantes (nacres), les glitters ou les particules métalliques d'aluminium ou autres.

En effet, les inventeurs ont découvert que l'utilisation de ces agents d'amélioration de la brillance dans une formulation de vernis à ongles permettait d'en améliorer la brillance, en réduisant l'effet négatif sur la brillance surtout des composants colorants comme les pigments.

Parmi les pigments utilisables dans l'invention on citera le dioxyde de titane (CI 77891), l'oxyde de fer noir (CI 77499), l'oxyde de fer rouge (CI 77491), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Les particules métalliques sont préparées par un procédé permettant d'obtenir des particules fines et plates pour une meilleure réflexion.

De préférence, ces compositions comprenant en outre un ou plusieurs autres additifs choisis parmi les agents filmogènes autres que ceux dérivés de la cellulose, les résines, les plastifiants, les agents rhéologiques, les absorbants UV, les modificateurs de surface, les agents de soins ou agents dits « traitants », etc.

Les agents filmogènes sont sélectionnés pour leur aptitude à former un film poreux après séchage (évaporation partielle ou totale du ou des solvants), de préférence parmi les dérivés de cellulose. Les différents filmogènes dérivés de cellulose utilisables dans l'invention sont les suivants :
- la nitrocellulose ayant un taux d'azote compris entre 11.8 et 12.3, il s'agit d'un grade de nitrocellulose soluble dans les solvants de type ester.
- La nitrocellulose ayant un taux d'azote inférieur 11.8, il s'agit d'un grade de nitrocellulose soluble dans les solvants de type alcool.

La nitrocellulose se présente sous la forme d'une poudre blanche humide. La nitrocellulose est en effet commercialisée sous forme « mouillée » (ou « damped ») avec plusieurs types de solvant comme l'éthanol, l'isopropanol ou encore l'eau.

D'autres agents filmogènes dérivés de cellulose utilisables sont :
- La cellulose acétate butyrate. Il existe de nombreux grades de cet agent filmogène qui se différencient par le taux d'estérification de la cellulose mais aussi par le rapport du nombre de fonction ester acétate et du nombre de fonction ester butyrate. Comme pour la nitrocellulose, la cellulose acétate butyrate peut également se différencier par la longueur du polymère cellulose utilisé pour produire le filmogène.
- L'éthylcellulose de différents grades de viscosité.
- L'hydroxypropylcellulose de différents grades de viscosité.

Les agents filmogènes sont utilisés seuls ou sous la forme de mélange dans la composition dans des proportions allant de 5% à 25% en poids, de préférence 6 à 20 % en poids, en particulier de 7,5 à 15% en poids de la composition totale.

Les résines permettent de donner du collant et de l'adhérence au film sur l'ongle. Les résines pouvant être utilisées sont les suivantes :
- La résine tosylamide/formaldéhyde (résine toluènesulfonamide/formaldéhyde) vendue sous la dénomination commerciale Ketjenflex MH, Ketjenflex MS-80 par Akzo Nobel ou encore Sulfonex par Estron.
- La résine tosylamide/époxy (résine toluènesulfonamide/époxy) vendue sous la dénomination commerciale de Lustrabrite S ou Lustrabrite S-70 ou Nagellite 3050 par Telechemische ou encore sous la dénomination polytex resin par Estron.
- Copolymère acide adipique/néopentyl glycol/anhydride trimellitique vendu sous la dénomination commerciale Uniplex 670-P par Unitex ou encore Chemol 509-9514 par Cook Composites
- Copolymère d'anhydride phtalique/glycérine/glycidyl décanoate
- Copolymère d'anhydride phtalique/anhydride trimellitique/glycols vendu sous la dénomination commerciale Polynex Resin par Estron ou encore 7809 Polynex Resin par Degen
- Copolymère de glycérine/acide phtalique
- Copolymère styrène/acrylate/acrylonitrile
- Polymères et copolymères d'acrylates
- Copolymères d'acrylates et de styrène
- Sucrose acétate isobutyrate vendu sous la dénomination commerciale SAIB par Eastman
- Résine polyvinylbutyral

En plus de leur action collante, la plupart de ces résines ont également une action plastifiante. Les résines peuvent être utilisées dans la formule seules ou sous la forme de mélanges dans des quantités allant de 0.2 à 15% en poids, de préférence 0.25 à 12 % en poids, en particulier de 0.5 à 10 % en poids de la composition totale.

Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 2 à 10 % en poids, de préférence 2,5 à 7,5 % en poids, en particulier de 3 à 6 % en poids de la composition totale.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate, etc.Différents additifs sont utilisables dans l'invention :

Parmi les additifs de rhéologie, on citera notamment les dérivés d'argile modifiés comme le stéaralkonium hectorite ou le stéaralkonium bentonite. Ces additifs de rhéologie seront de préférence utilisés de 0.1 à 3 % en poids total de la formulation. Ces additifs permettent notamment la suspension des particules insolubles dans le vernis tels que les pigments ou encore les nacres.

Parmi les absorbants UV, on citera les absorbants de lumière UV qui permettent de protéger la formulation ou les agents colorants utilisés l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller de 0.1 à 1 % en poids.

Parmi les additifs utilisables selon l'invention, on citera également les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0.01 à 0.5 % en poids total de la formulation.

On citera également les additifs dits « traitants » de l'ongle utilisables dans la composition selon l'invention. Les additifs traitants peuvent représenter de 0.0001% à 5%, de préférence de 0.01% à 1% en poids de la composition totale. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane vendu sous la dénomination commerciale Pro-DSB par Exsymol,
- la myrrhe,
- les acides aminés soufrés comme la cystéine, ses sels et leurs dérivés, la cystine, le glutathion. Les acides aminés soufrés sont en effet capables de créer des cross-linkings entre la kératine et les groupes SH libres de ces dérivés soufrés,
- la kératine hydrolysée d'origine végétale comme le Prosina de Croda,
- les alpha-hydroxyacides comme l'acide citrique, l'acide ascorbique,
- la biotine,
- l'urée et la diméthylurée.

Dans un aspect supplémentaire, l'invention propose donc l'utilisation d'un un ou de plusieurs copolymères obtenus par condensation d'acide adipique et de pentaérythritol comprenant des groupes terminaux d'acide caproïque, d'acide heptanoïque, d'acide caprylique et/ou d'acide caprique comme agent(s) d'amélioration de la brillance dans la préparation de compositions cosmétiques. De préférence, la quantité de copolymère(s) par condensation d'acide adipique et de pentaérythritol comprenant des groupes terminaux d'acide caproïque, d'acide heptanoïque, d'acide caprylique et/ou d'acide caprique représente de 0.01 à 25 % en poids de la composition cosmétique totale.

L'invention concerne également un procédé de préparation d'une telle composition cosmétique anhydre, notamment pour vernis à ongles ou de soins des ongles, dans lequel on mélange au moins un solvant organique cosmétiquement acceptable, une quantité efficace d'au moins un agent d'amélioration de la brillance choisi parmi les copolymères par condensation d'acide adipique et de pentaérythritol comprenant des groupes terminaux d'acide caproïque, d'acide heptanoïque, d'acide caprylique et/ou d'acide caprique ou leurs mélanges, et de préférence au moins un agent de coloration choisi parmi les pigments, les nacres, les glitters et/ou les particules métalliques, ainsi qu'éventuellement un ou plusieurs autres additifs choisis parmi les agents filmogènes, les résines, les plastifiants, les agents rhéologiques, les absorbants UV et les modificateurs de surface, les agents dits « traitants », etc.

Un avantage supplémentaire de l'invention est que les agents d'amélioration de la brillance peuvent non seulement être ajoutés à n'importe quel moment du procédé, c'est-à-dire que l'effet des présents agents n'est pas affecté par l'ordre dans lequel les ingrédients sont dosés. De plus, en complément des avantages déjà mentionnés ci-dessus en relation avec les compositions, il est à remarquer que le procédé ci-dessus peut être réalisé moyennant des installations tout à fait standard et ne demande pas de connaissances, ni de précautions particulières de l'opérateur.

En conclusion, les inventeurs ont découvert d'une manière surprenante que lesdits copolymères par condensation d'acide adipique et de pentaérythritol comprenant des groupes terminaux d'acide caproïque, d'acide heptanoïque, d'acide caprylique et/ou d'acide caprique ou leurs mélanges, permettaient de réaliser l'objectif d'augmenter nettement la brillance de compositions cosmétiques anhydres après application et de la maintenir pendant plus longtemps.

### Exemples

Ci-dessous, quelques exemples de formulations de dispersions colorantes comparatives montrant l'amélioration de la brillance.

La préparation de vernis à ongles se déroule généralement en deux étapes.

La première consiste à préparer la base, il s'agit en fait d'un vernis à ongles mais ne contenant pas d'ingrédient colorant. La base se présente sous la forme d'un liquide opaque à laquelle sont ajoutés dans une deuxième étape les pigments, nacres ainsi que les autres composants susceptibles d'apporter de la couleur à la formulation.

Il est généralement clairement noté une diminution de la brillance entre la base sans couleur et la composition finale colorée.

Nous avons pris une dispersion colorante et y avons ajouté un pourcentage soit de Polyester-4, soit de base servant à la dispersion. De cette façon les taux de pigments sont identiques.

La méthode pour déterminer la brillance du film formé est réalisée de la manière suivante :

Le vernis coloré est appliqué sur une carte normalisée à l'aide d'un applicateur permettant la formation d'un film humide d'une épaisseur de 100 mm. Le film est laissé séché pendant 15 minutes à température ambiante. La brillance est mesurée à l'aide d'un brillancemètre sous un angle de 60° par rapport à la normale du film. La brillance est notée en % car elle correspond en fait au pourcentage de lumière réfléchie par rapport à la lumière incidente.

Les formulations de l'Ex 1 et l'Ex 8 dans les Tableaux 1 et 2 ci-dessous sont données à titre comparatif et représentent une composition connue de l'état de la technique.

Les brillances instantanées des 2 échantillons obtenus sont comparées, soit Ex 2 comparé à Ex 3, Ex 4 à Ex 5, et Ex 6 à Ex 7, etc.

**Tableau 1 : Exemples 1 à 7 (Red 6 Ba lake)**

| **Ingrédients** | **Ex 1** | **Ex 2** | **Ex 3** | **Ex 4** | **Ex 5** | **Ex 6** | **Ex 7** |
|---|---|---|---|---|---|---|---|
| Acétate d'éthyle | 25.52 | 25.52 | 25.52 | 25.52 | 25.52 | 25.52 | 25.52 |
| Acétate de butyle | 24.64 | 24.64 | 24.64 | 24.64 | 24.64 | 24.64 | 24.64 |
| Nitrocellulose (70% Isopropanol) | 17.60 | 17.60 | 17.60 | 17.60 | 17.60 | 17.60 | 17.60 |
| Résine polyester | 12.32 | 12.32 | 12.32 | 12.32 | 12.32 | 12.32 | 12.32 |
| Copolymère styrène/acrylique | 1.76 | 1.76 | 1.76 | 1.76 | 1.76 | 1.76 | 1.76 |
| Acétyl tributyl citrate | 6.16 | 6.16 | 6.16 | 6.16 | 6.16 | 6.16 | 6.16 |
| DC Red 6 Ba Lake | 12 | 12 | 12 | 12 | 12 | 12 | 12 |
| | | | | | | | |
| Polyester-4 | 0 | 5 | 0 | 10 | 0 | 20 | 0 |
| Base pour Dilution | 0 | 0 | 5 | 0 | 10 | 0 | 20 |
| | | | | | | | |
| | | | | | | | |
| Total | 100 | 105 | 105 | 110 | 110 | 120 | 120 |
| Brillance | 66 | 70 | 67 | 78 | 69 | 82 | 72 |

**Tableau 2 : Exemples 8 à 14 (Red 7 Ca lake)**

| **Ingrédients** | **Ex 8** | **Ex 9** | **Ex 10** | **Ex 11** | **Ex 12** | **Ex 13** | **Ex 14** |
|---|---|---|---|---|---|---|---|
| Acétate d'éthyle | 26.39 | 26.39 | 26.39 | 26.39 | 26.39 | 26.39 | 26.39 |
| Acétate de butyle | 25.48 | 25.48 | 25.48 | 25.48 | 25.48 | 25.48 | 25.48 |
| Nitrocellulose (70% Isopropanol) | 18.2 | 18.2 | 18.2 | 18.2 | 18.2 | 18.2 | 18.2 |
| Résine polyester | 12.74 | 12.74 | 12.74 | 12.74 | 12.74 | 12.74 | 12.74 |
| Copolymère styrène/acrylique | 1.82 | 1.82 | 1.82 | 1.82 | 1.82 | 1.82 | 1.82 |
| Acétyl tributyl citrate | 6.37 | 6.37 | 6.37 | 6.37 | 6.37 | 6.37 | 6.37 |
| DC Red 7 Ca lake | 9.00 | 9.00 | 9.00 | 9.00 | 9.00 | 9.00 | 9.00 |
| | | | | | | | |
| Polyester-4 | 0 | 5 | 0 | 10 | 0 | 20 | 0 |
| Base pour Dilution | 0 | 0 | 5 | 0 | 10 | 0 | 20 |
| | | | | | | | |
| Total | 100 | 105 | 105 | 110 | 110 | 120 | 120 |
| Brillance | 35 | 37 | 36 | 41 | 38 | 55 | 42 |

Nous pouvons remarquer que l'augmentation de la brillance de la solution colorante provient bien de l'utilisation de Polyester-4 et non pas de la dilution.

En résumé, l'inventeur a découvert que l'utilisation d'un agent d'augmentation de la brillance selon l'invention, comme le Polyester-4, permettait d'une part de diminuer la différence de brillance entre la base et la composition finale colorée, et d'autre part de maintenir ce niveau de brillance pendant un temps plus long que celui observé habituellement.

L'ajout de l'agent d'augmentation de la brillance selon l'invention est de préférence réalisé au moment de la fabrication de la solution colorante. Il a clairement été noté que l'agent d'augmentation de la brillance selon l'invention a une plus grande influence sur la brillance instantanée et à plus long terme de la formulation dans des vernis à ongles contenant des pigments ou contenant des nacres en faible quantité, la brillance de teintes contenant uniquement des nacres étant plus faiblement augmentée.

## Revendications

1. Composition cosmétique anhydre pour vernis à ongles, comprenant au moins un solvant organique cosmétiquement acceptable, au moins un agent filmogène dérivé de la cellulose et une quantité efficace d'au moins un agent d'amélioration de la brillance, **caractérisée en ce que** le au moins un agent d'amélioration de la brillance est un copolymère par condensation d'acide adipique et de pentaérythritol comprenant des groupes terminaux d'acide caproïque, d'acide heptanoïque, d'acide caprylique et/ou d'acide caprique ou un mélange de plusieurs de ces copolymères.

2. Composition selon la revendication 1, dans laquelle le au moins un agent d'amélioration de la brillance est le polyester-4.

3. Composition selon la revendication 1 ou 2, dans laquelle le au moins un agent d'amélioration de la brillance représente 0.01 à 25 % en poids, de préférence 0.1 à 20 % en poids, en particulier de 0.25 à 15 % en poids de la composition totale.

4. Composition selon l'une quelconque des revendications 1 à 3, comprenant en outre de 0.1 à 10 % en poids de la composition totale d'un ou de plusieurs agents de coloration choisis parmi les pigments, les nacres, les glitters et/ou les particules métalliques.

5. Composition selon l'une quelconque des revendications précédentes, comprenant en outre un ou plusieurs autres additifs choisis parmi les agents filmogènes autres que ceux dérivés de la cellulose, les résines, les plastifiants, les agents rhéologiques, les absorbants UV, les modificateurs de surface et les agents dits « traitants ».

6. Utilisation d'un copolymère par condensation d'acide adipique et de pentaérythritol comprenant des groupes terminaux d'acide caproïque, d'acide heptanoïque, d'acide caprylique et/ou d'acide caprique ou d'un mélange de plusieurs de ces copolymères dans la préparation d'une composition cosmétique anhydre pour vernis à ongles comprenant au moins un agent filmogène dérivé de la cellulose, comme agent(s) d'amélioration de la brillance.

7. Utilisation selon la revendication 6, dans laquelle la quantité de copolymère(s) par condensation d'acide adipique et de pentaérythritol comprenant des groupes terminaux d'acide caproïque, d'acide heptanoïque, d'acide caprylique et/ou d'acide caprique représente 0.01 à 25 % en poids de la composition cosmétique totale.

8. Procédé de préparation d'une composition cosmétique anhydre pour vernis à ongles selon l'une quelconque des revendications 1 à 5, comprenant le mélange d'au moins un solvant organique cosmétiquement acceptable, d'au moins un agent filmogène dérivé de la cellulose, d'au moins un agent d'amélioration de la brillance choisi parmi les copolymères par condensation d'acide adipique et de pentaérythritol comprenant des groupes terminaux d'acide caproïque, d'acide heptanoïque, d'acide caprylique et/ou d'acide caprique ou leurs mélanges.

9. Procédé selon la revendication 8, comprenant en outre l'incorporation dans le mélange d'au moins un agent de coloration choisi parmi les pigments, les nacres les glitters et/ou les particules métalliques.

10. Procédé selon la revendication 8 ou 9, comprenant en outre l'incorporation dans le mélange d'un ou de plusieurs autres additifs choisis parmi les agents filmogènes autres que ceux dérivés de la cellulose, les résines, les plastifiants, les agents rhéologiques, les absorbants UV, les modificateurs de surface et les agents dits « traitants ».

11. Procédé selon l'une quelconque des revendications 8, 9 ou 10, dans lequel la quantité de copolymère(s) par condensation d'acide adipique et de pentaérythritol comprenant des groupes terminaux d'acide caproïque, d'acide heptanoïque, d'acide caprylique et/ou d'acide caprique représente 0.01 à 25 % en poids de la composition cosmétique totale.

## Patentansprüche

1. Wasserfreie kosmetische Zusammensetzung für Nagellacke, die mindestens ein kosmetisch akzeptables organisches Lösemittel, mindestens einen Cellulosederivat-Filmbildner und eine wirksame Menge von mindestens einem Mittel zur Verbesserung des Glanzes enthält, **dadurch gekennzeichnet, dass** das mindestens eine Mittel zur Verbesserung des Glanzes ein durch Kondensation von Adipinsäure und Pentaerythrit entstandenes Copolymer, das Endgruppen aus Capronsäure, Önanthsäure, Caprylsäure und/oder Caprinsäure umfasst, oder ein Gemisch mehrerer dieser Copolymere ist.

2. Zusammensetzung gemäß Anspruch 1, bei der das mindestens eine Mittel zur Verbesserung des Glanzes Polyester-4 ist.

3. Zusammensetzung gemäß Anspruch 1 oder 2, bei der das mindestens eine Mittel zur Verbesserung des Glanzes einen Massenanteil von 0,01 bis 25 %, vorzugsweise einen Massenanteil von 0,1 bis 20 % und im Besonderen einen Massenanteil von 0,25 bis 15 % der gesamten Zusammensetzung darstellt.

4. Zusammensetzung gemäß einem beliebigen der Ansprüche von 1 bis 3, die außerdem ein oder mehrere aus Pigmenten, Perlmutt, Glitter und/oder Metallteilchen ausgewählte/s Färbemittel in einem Massenanteil von 0,1 bis 10 % der gesamten Zusammensetzung umfasst.

5. Zusammensetzung gemäß einem beliebigen der vorhergehenden Ansprüche, die außerdem einen oder mehrere Zusatzstoff/e umfasst, die aus anderen filmbildenden Mitteln als Cellulosederivat-Filmbildnern, Harzen, Weichmachern, rheologischen Mitteln, UV-Absorbern, Mitteln zur Oberflächenmodifizierung und sogenannten "Pflegemitteln" ausgewählt sind.

6. Verwendung eines durch Kondensation von Adipinsäure und Pentaerythrit entstandenen Copolymers, das Endgruppen aus Capronsäure, Önanthsäure, Caprylsäure und/oder Caprinsäure umfasst, oder eines Gemischs mehrerer dieser Copolymere bei der Zubereitung einer wasserfreien kosmetischen Zusammensetzung für Nagellacke, die mindestens einen Cellulosederivat-Filmbildner als Mittel zur Verbesserung des Glanzes umfasst.

7. Verwendung gemäß Anspruch 6, bei der die Menge des/der durch Kondensation von Adipinsäure und Pentaerythrit entstandenen Copolymers/e, das/die Endgruppen aus Capronsäure, Önanthsäure, Caprylsäure und/oder Caprinsäure umfasst/umfassen, einen Massenanteil von 0,01 bis 25 % der gesamten kosmetischen Zusammensetzung darstellt.

8. Verfahren zur Zubereitung einer wasserfreien kosmetischen Zusammensetzung für Nagellacke gemäß einem beliebigen der Ansprüche von 1 bis 5, das die Mischung von mindestens einem kosmetisch akzeptablen organischen Lösemittel, von mindestens einem Cellulosederivat-Filmbildner, von mindestens einem Mittel zur Verbesserung des Glanzes umfasst, das aus durch Kondensation von Adipinsäure und Pentaerythrit entstandenen Copolymeren, die Endgruppen aus Capronsäure, Önanthsäure, Caprylsäure und/oder Caprinsäure umfassen, oder deren Gemischen ausgewählt ist.

9. Verfahren gemäß Anspruch 8, das außerdem die Beimengung von mindestens einem Färbemittel in das Gemisch umfasst, das aus Pigmenten, Perlmutt, Glitter und/oder Metallteilchen ausgewählt ist.

10. Verfahren gemäß Anspruch 8 oder 9, das außerdem die Beimengung von einem oder mehreren Zusatzstoffen in das Gemisch umfasst, der/die unter anderen filmbildenden Mitteln als Cellulosederivat-Filmbildnern, Harzen, Weichmachern, rheologischen Mitteln, UV-Absorbern, Mitteln zur Oberflächenmodifizierung und sogenannten "Pflegemitteln" ausgewählt ist/sind.

11. Verfahren gemäß einem beliebigen der Ansprüche 8, 9 oder 10, bei dem die Menge des/der durch Kondensation von Adipinsäure und Pentaerythrit entstandenen Copolymers/e, das/die Endgruppen aus Capronsäure, Önanthsäure, Caprylsäure und/oder Caprinsäure umfasst/umfassen, einen Massenanteil von 0,01 bis 25 % der gesamten kosmetischen Zusammensetzung darstellt.

## Claims

1. An anhydrous cosmetic composition for nail varnish, comprising at least one cosmetically acceptable organic solvent, at least one film-forming agent derived from cellulose and an effective quantity of at least one gloss-enhancing agent, **characterised in that** the at least one gloss-enhancing agent is a condensation copolymer of adipic acid and pentaerythritol comprising caproic acid, heptanoic acid, caprylic acid and/or capric acid end groups or a mixture of a plurality of these copolymers.

2. A composition according to claim 1, in which the at least one gloss-enhancing agent is Polyester-4.

3. A composition according to claim 1 or 2, in which the at least one gloss-enhancing agent represents 0.01 to 25% by weight, preferably 0.1 to 20% by weight, in particular 0.25 to 15% by weight of the total composition.

4. A composition according to any one of claims 1 to 3, furthermore comprising from 0.1 to 10% by weight of the total composition of one or more colouring agents selected from pigments, pearlescents, glitters and/or metallic particles.

5. A composition according to any one of the preceding claims, furthermore comprising one or more other additives selected from film-forming agents other than those derived from cellulose, resins, plasticisers, rheological agents, UV absorbents, surface modifiers and "treatment" agents.

6. Use of a condensation copolymer of adipic acid and pentaerythritol comprising caproic acid, heptanoic acid, caprylic acid and/or capric acid end groups or a mixture of a plurality of these copolymers in the preparation of an anhydrous cosmetic composition for nail varnish comprising at least one film-forming agent derived from cellulose, as gloss-enhancing agent(s).

7. Use according to claim 6, in which the quantity of condensation copolymer(s) of adipic acid and pentaerythritol comprising caproic acid, heptanoic acid, caprylic acid and/or capric acid end groups represents 0.01 to 25% by weight of the total cosmetic composition.

8. A method of preparing an anhydrous cosmetic composition for nail varnish according to any one of claims 1 to 5, comprising mixing at least one cosmetically acceptable organic solvent, at least one film-forming agent derived from cellulose, and at least one gloss-enhancing agent selected from condensation copolymers of adipic acid and pentaerythritol comprising caproic acid, heptanoic acid, caprylic acid and/or capric acid end groups or mixtures thereof.

9. A method according to claim 8, furthermore comprising incorporating at least one colouring agent selected from pigments, pearlescents, glitters and/or metallic particles into the mixture.

10. A method according to claim 8 or 9, furthermore comprising incorporating one or more other additives selected from film-forming agents other than those derived from cellulose, resins, plasticisers, rheological agents, UV absorbents, surface modifiers and "treatment" agents into the mixture.

11. A method according to any one of claims 8, 9 or 10, in which the quantity of condensation copolymer(s) of adipic acid and pentaerythritol comprising caproic acid, heptanoic acid, caprylic acid and/or capric acid end groups represents 0.01 to 25% by weight of the total cosmetic composition.
